# EUROPEAN PATENT SPECIFICATION

(11) **EP 4 154 179 B1**
(45) Date of publication and mention of the grant of the patent: **18.12.2024**
(21) Application number: 21722267.8
(22) Date of filing: 05.05.2021
(51) Int. Cl.: G06K 19/077, A61M 5/32

(54) **RING-SHAPED RFID LABEL FOR SYRINGE TIP CAP**
RINGFÖRMIGES RFID-ETIKETT FÜR EINE SPRITZENSPITZENKAPPE
ÉTIQUETTE RFID EN FORME D'ANNEAU POUR UN CAPUCHON DE POINTE DE SERINGUE

(30) Priority: 18.05.2020 EP 20175265
(43) Date of publication of application: 29.03.2023
(73) Proprietor: Becton Dickinson France, 38800 Le Pont-de-Claix (FR); Becton Dickinson and Company, Franklin Lakes, NJ 07417-1880 (US)
(72) Inventor: RIVIER, Cédric, 38340 VOREPPE (FR); EUVRARD, Nicolas, Plainsboro NJ 08536 (US); LEIBBRAND, Alfred, 38640 Claix, Isère (FR); MARTY, Daniel, 8640 Rapperswil (CH); THOMAS, Fassler, 8627 Grüningen (CH)
(74) Representative: Germain Maureau
(86) International application number: PCT/EP2021/061883
(87) International publication number: WO 2021/233684

(56) References cited:
- WO-A1-2019/222139
- WO-A1-2020/260298
- US-A1- 2004 220 602
- US-A1- 2017 172 701
- US-A1- 2019 217 018

## Description

The present invention relates to a cover for a medical injection device, a medical injection device and a method for manufacturing said cover. The invention is particularly well suited for the healthcare industry.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to a medical container of the invention, and the "proximal direction" is to be understood as meaning the opposite direction to said direction of injection, that is to say the direction towards the user's hand holding a container as for an injection operation.

Medical injection devices, for example pre-fillable or prefilled syringes, usually comprise a hollow body or barrel forming a container for a medical product. This body comprises a distal end, optionally provided with a needle, and a proximal end, usually provided with a flange.

There is an increasing need for individual traceability of the medical containers, such as medical injection devices, from the manufacturing process until the final labeling, the final use or the disposal of said medical containers.

It is known, for example, from WO2017157784 a receptacle having a cylindrical lateral surface surrounded by a sequence of printed machine-readable unique identifier codes. These printed unique identifier codes allow tracking and tracing of each receptacle along a supply chain. However, these unique identifier codes are printed on an external side of the receptacle so that they may be removed or damaged, for example, during handling or use of the receptacle. Moreover, the unique identifier codes cover a portion of the receptacle so that they may have an impact on a user visual inspection process. Finally, an inkjet printer is used to print the identifier codes on the external side of the receptacle. However, this printing method, using ink, may lead to a risk of contamination of the receptacle. Moreover, one may not have access to these printed unique identifier codes when the receptacle is put for example in a sealed packaging.

It is further known from EP2019305879, a plastic flange for a medical container, said flange comprising a remotely readable electronic component such as RFID tag including a RFID chip and a RFID antenna for remote identification of the medical container. However, said medical container requires a complex manufacturing process which includes in particular the assembly of a plastic flange on a glass barrel.

US2019/0217018 discloses a RFID tag enabled shield assembly that provides a sterile enclosure of a medicament delivery port of a medicament container. The container can be a needleless pre-filled syringe, a vial, a cartridge, or a collapsible bag or pouch. The RFID tag is fixedly attached to one or more components of the shield assembly through co-molding or another form of permanent or removable attachment. The RFID tag is actually a RFID chip that can be optionally in electrical communication with an antenna. In one embodiment, shield assemblies comprise a two part less flexible component such as a hub and a removable top. The removable top can contain and hold the flexible component. There can be a headspace located above the terminal end face of the flexible component and the distal inside surface of the top. The flexible component can contain an RFID tag. Nevertheless, the presence of the RFID tag can inhibit the sterilization process. Usually, a sterilization gas goes through the shield assembly from the top of the shield assembly. The RFID tag in US2019/0217018 blocks the entry through which the sterilization gas flows.

In this context, an object of the present invention is to provide a device that alleviates the above-mentioned drawbacks by allowing a long range individual identification of a medical injection device with no impact on visual inspection, with few or no risks of being removed or damaged, with no risk to inhibit the sterilization process and with a limited impact on the manufacturing process.

A first aspect of the present invention is a cover for a medical injection device, said medical injection device having a hub portion defined at its distal end, the said cover comprising:
- an outer casing formed of a first material, said outer casing having a distal end and a proximal end,
- an inner casing formed of a second material different from the said first material and defining a cavity capable of receiving in a sealing way, at least part of the hub portion, said inner casing having a distal end and a proximal end, and being at least partly inserted within the outer casing and at least partially in contact with the outer casing and
- at least one substantially ring-shaped Radio Frequency Identification (RFID) tag embedded within the cover, said ring-shaped RFID tag comprising a RFID chip connected to at least one ring-shaped RFID antenna.

Without willing to be bound by any theory, it is believed that the cover of the invention allows an optimized individual traceability of each medical injection device from the manufacturing process to the final use of the medical injection device. Besides, the ring-shaped RFID tag is well protected from removal or external damage that may occur due to the packaging, storing distribution or the use of the medical injection device. Furthermore, the ring-shaped RFID tag being embedded within the cover, there is no visual impact on the medical injection device. Additionally, the insertion of the ring-shaped RFID tag has only a limited impact on the cover manufacturing process thanks to the shape of the RFID tag. It is also contemplated that the ring-shaped RFID tag allows remote and therefore easy and longue distance identification of the medical injection device, from the manufacturing steps of the cover to the final use of the medical injection device or its disposal. Another advantage of the invention is that the RFID tag does not require a direct visual perspective from a reading machine so that the reading may occur at any time without a need to unpack the medical injection device, the medical injection device being packed in an individual packaging or packed with others medical injection devices such as in a tub and/or a sealing bag. Moreover, there is no additional thickness to an outer wall of the cover, and thus no change is required regarding the packaging or storing of the medical injection device.
According to the present invention, the ring-shaped Radio Frequency Identification (RFID) tag is due to the at least one RFID antenna having mainly a substantially ring shape. Preferably, the RFID chip has a substantially retangular or square shape. Thus, the specific ring-shaped of the RFID tag combined with the radial dimensions of the RFID tag with respect to the cover and the specific position of the RFID tag does not inhibit, during a sterilization step, the sterilization gas such as ethylene oxide or water vapor going through the outer casing of the cover, said outer casing being impermeable to the sterilization gas. Additionally, the at least one antenna may be as long as desired within a ring conformation. This makes it possible to include a RFID tag in a cover without increasing the overall cover volume. Ultimately this allows increasing data transmission level to the RFID reader. Indeed the longer the antenna, the better the data transmission.

The inner casing of the cover of the present invention comprises an outer wall being at least partly in contact with the inner wall of the outer casing. The contact is made without any adhesive. Preferably, the outer wall of the inner casing is only partly in contact with the inner wall of the outer casing. In this embodiment, the inner and outer casings can be easily assembled together.

Preferably, the first material forming the outer casing of the cover of the invention is more rigid than the second material forming the inner casing of the cover of the invention.

Preferably, said first material is a thermoplastic, and may be polypropylene (PP), polyethylene (PE), polyethylene terephthalate (PET), polystyrene (PS) or polycarbonate (PC).

Preferably, the second material is a deformable material, made of a material having elastomeric properties, such as Thermo Plastic Elastomer ("TPE"), rubber or elastomer. Materials with elastomeric properties that are sterilizable are preferred.

Advantageously, the cover of the present invention comprises one substantially ring-shaped RFID tag. The ring-shaped RFID tag is embedded within the cover. In other words, the ring-shaped RFID tag is inextricably linked to the cover, and the ring-shaped RFID tag may not be removed from the cover. In the present invention, "embedded" means that the ring-shaped RFID tag is overmolded within the cover or that the ring-shaped RFID that is implemented within the cover by adhesive bonding or by assembly.

In one embodiment, the ring-shaped RFID tag is located at the distal ends of the outer and inner casings.

In another embodiment, the ring-shaped RFID tag is located at a middle portion of the outer casing.

In another embodiment, the ring-shaped RFID tag is located at a proximal of the outer and inner casings.

In one embodiment, the ring-shaped RFID tag is located on a top inner wall of the outer casing or on a top outer wall of the inner casing. Preferably, the ring-shaped RFID tag is located on a top inner wall of the outer casing.

In one embodiment, the ring-shaped RFID tag is embedded within the outer casing, especially between a lateral inner wall of the outer casing and a lateral outer wall of the outer casing.

In one emdobiment, the ring-shaped RFID tag is embedded within the outer casing and is flush with a lateral inner wall of the outer casing. Preferably, the ring-shaped RFID tag is not embedded within the external surface of the cover, in particular the ring-shaped RFID tag is not embedded within the outer casing.

In one embodiment, the ring-shaped RFID tag embedded in the cover of the invention is in a form of wet inlay, dry inlay, or pressure sensitive label.

RFID tags, and particularly RFID Wet Inlays and RFID Dry Inlays, can comprise a substrate, for example made of paper or Polyethylene terephthalate (PET). The RFID tag can be disposed on one side of the substrate. RFID Wet Inlays and RFID Dry Inlays can further comprise at least one protective layer on top of the RFID tag. The protective layer can be a siliconized paper.

RFID Wet Inlays are described as "wet" as they include an adhesive layer on the other side of the substrate and a backing paper, for example with a silicon liner. RFID Dry Inlays are described as "Dry" due to their lack of adhesive backing. Pressure-sensitive labels are analogous to a high-tech sticker.

In one embodiment, the ring-shaped RFID tag is a Low Frequency Radio Frequency Identification (LF-RFID) tag. Low frequencies are usually about 30 KHz to 300 KHz. In this embodiment, a RFID reader can for example read the LF-RFID tag at a distance up to about 10 cm.

In one embodiment, the ring-shaped RFID tag is a High Frequency Radio Frequency Identification (HF-RFID) tag. High frequencies are usually about 3-30 MHz. In this embodiment, a RFID reader can for example read the HF-RFID tag at a distance about one meter.

In one embodiment, the ring-shaped RFID tag is a High-Frequency Near Field Communication (HF-NFC) tag. The frequency is usually about 13.56 MHz. In this embodiment, a NFC reader can for example read the HF-NFC tag at a distance up to a few centimeters. HF-NFC differs from HF-RFID in that it can be read by a NFC smartphone.

In one embodiment, the ring-shaped RFID tag is a double frequency tag including simultaneously a HF-NFC and an UHF RFID. For example, it can be read with both a NFC smartphone or an UHF reader.

Preferably, the ring-shaped RFID tag is an Ultra High Frequency Radio Frequency Identification (UHF-RFID) tag. Ultra high frequencies are usually about 400-1000MHz. In this embodiment, a RFID reader can for example read the UHF-RFID tag at a distance about fifteen meters. A ring-shaped RFID tag means that the at least one antenna has substantially ring shape.

In one embodiment, the ring-shaped RFID tag comprises at least one antenna having two extremities, each extremity being linked to a portion of the RFID chip. In this embodiment, the RFID tag comprises a RFID chip and at least one antenna having a substantially ring shape.

In another embodiment, the ring-shaped RFID tag comprises at least one antenna having two legs including respectively a first extremity and a second extremity, each first extremity of the legs being linked to a portion of the RFID chip. Preferably, the second extremities of the legs of the RFID antenna are not in contact with each other. It is believed that in these embodiments, the shape of the legs further improves the communication between the chip, the antenna of the RFID tag and the RFID reader.

In another embodiment, the ring-shaped RFID tag comprises at least one antenna having two legs including respectively a first extremity and a second extremity, each first extremity of the legs being linked to a portion of the RFID chip and each second extremity including a plurality of sub-legs. In this embodiment, the two legs are respectively in a form of a rake.

In one embodiment, the at least one RFID antenna has a linear form.

In other embodiment, the at least one RFID antenna is made of a plurality of steady steps. For example, the RFID antenna has two legs which may have a plurality of steady steps. For example, the steady step forms a square, triangle, rectangle or wave shape. Preferably, the plurality of steady steps have the same amplitude.

For example, the RFID antenna has two sinusoidal shaped legs, straight-shaped legs or coil-shaped legs. Preferably, when the RFID antenna has two sinusoidal shaped legs, both legs of the RFID antenna being made of a plurality of sinusoids. For example, when the RFID tag is an UHF-RFID tag, the two legs have sinusoidal shape. For example, when the RFID tag is a HF-RFID tag, the two legs have a coil shape. Indeed, it is believed that in these embodiments, the shape of the legs further improves the communication between the chip, the antenna of the RFID tag and the RFID reader.

Preferably, the RFID antenna forms a loop between the legs, the RFID chip being located between the loop and the legs of the RFID antenna.

In one embodiment, the RFID tag is dissymmetrical, i.e. the RFID chip is not located at equidistance of both RFID antenna extremities.

Preferably, the RFID chip is located at equidistance of both RFID antenna extremities.

In one embodiment, the ring-shaped RFID tag comprises at least one dipole antenna. A dipole antenna commonly consists of two identical conductive antennas such as metal wires or rods connected to the RFID chip.

A second aspect of the present invention is a medical injection device comprising a hub portion, and a cover according to the present invention.

A third aspect of the present invention is a method for manufacturing a cover comprising an inner casing and an outer casing according to the present invention, comprising:
A. Providing the inner casing and the outer casing, said inner casing defining a cavity capable of receiving in a sealing way at least part of a hub portion defined at the distal end of a medical injection device,
B. Providing the inner casing and/or the outer casing with at least one ring-shaped RFID tag, said ring-shaped RFID tag comprising a RFID chip connected to at least one ring-shaped RFID antenna and
C. Assembling the cover by inserting the inner casing within the outer casing so that the RFID tag is embedded within the cover.

In step A), the inner casing and the outer casing may be manufactured separately. For example, the inner casing and the outer casing can be manufactured separately by molding.

Preferably, in step B), the inner casing and/or outer casing is provided with a ring-shaped RFID tag by overmolding, by adhesive bonding or by assembly.

For example, the ring-shaped RFID tag may be embedded within a top inner wall of the outer casing or on a top outer wall of the inner casing by over-molding or by assembly. Preferably, the ring-shaped RFID tag may be embedded within a top inner wall of the outer casing or on a top outer wall of the inner casing by over-molding.

For example, when the ring-shaped RFID tag is embedded between the inner casing and the outer casing, the ring-shaped RFID tag is implemented between a top inner wall of the outer casing and a top outer wall of the inner casing. For example the ring-shaped RFID tag may adhere to the outer wall of the inner casing or to the inner wall of the outer casing by adhesive bonding or by assembly.

In step C), the inner casing and the outer casing can be assembled to form the cover of the invention so that the RFID tag is embedded within the cover.

The invention and advantages arising therefrom will clearly emerge from the detailed description that is given below with reference to the appended drawings as follows:
Figure 1 is a view of a medical injection device comprising a cover according to an embodiment of the present invention;
Figure 2 is an exploded view a medical injection device comprising a cover according to an embodiment of the present invention;
Figure 3 is an exploded view of the cover according to an embodiment of the present invention;
Figure 4 is a view in section of a medical injection device comprising a cover according to an embodiment of the present invention;
Figure 5 is a zoomed view of Figure 4;
Figure 6 is a RFID tag according to an embodiment of the present invention;
Figure 7 is a RFID tag according to another embodiment of the present invention;
Figure 8 is a transversal view of another medical injection device comprising a cover according to another embodiment of the present invention;
Figure 9 is an exploded view of the embodiment illustrated in Figure 8;
Figure 10 is an exploded view of the cover as illustrated in Figures 8 and 9;
Figure 11 is a view in section of the embodiment illustrated in Figures 8 to 10;
Figure 12 is a zoomed view of Figure 11;
Figure 13 is a view in section of another medical injection device equipped with a cover according to another embodiment of the present invention.

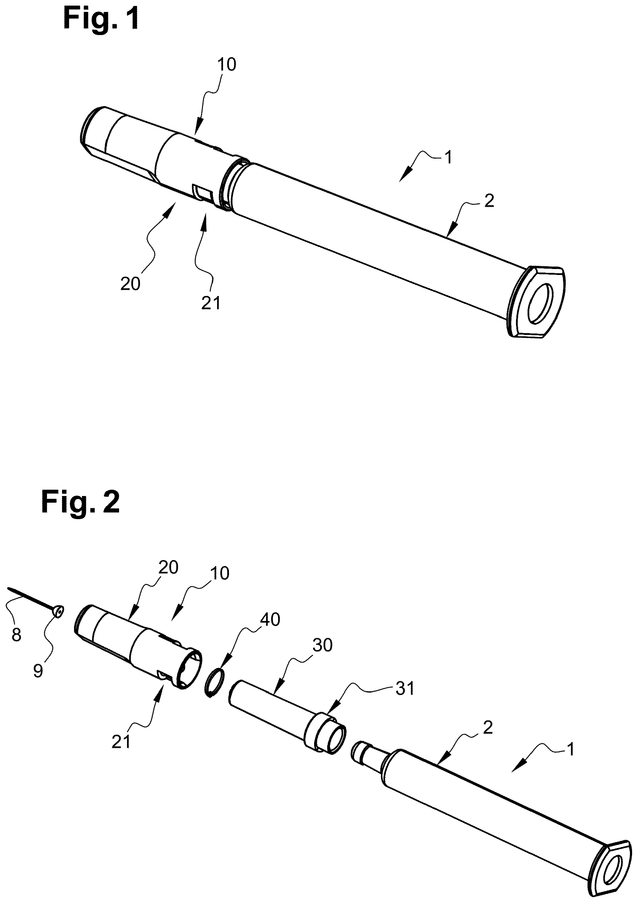

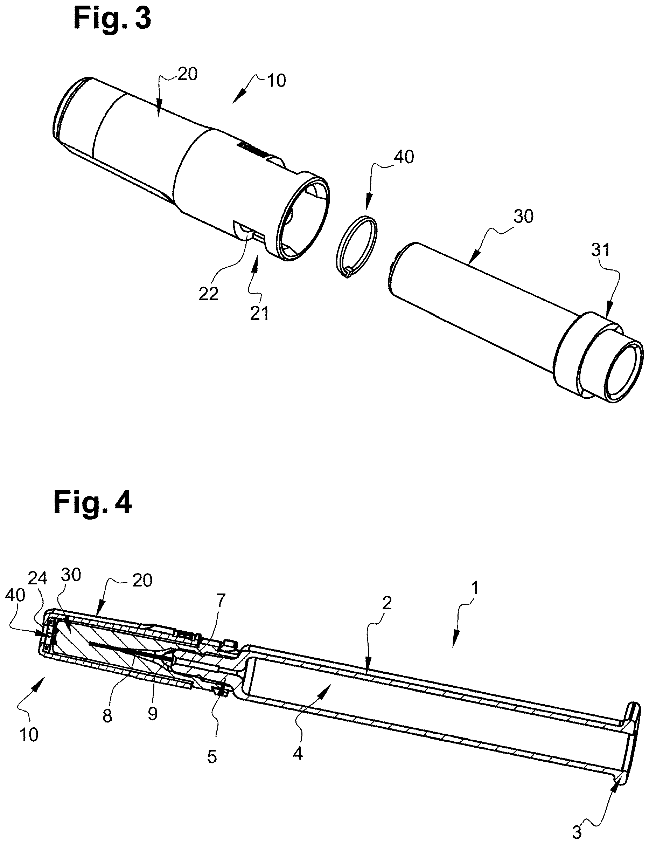

Figures 1, 2 and 4 show a medical injection device 1 such a syringe comprising a body 2. Said body 2 comprises a sidewall 3 forming a reservoir adapted to contain a medical composition 4 to be injected. The medical injection device 1 further comprises a hub portion 5 provided at its distal end 6. The hub portion 5 is partially hollow so as to form a channel 7 in fluidic communication with the body 2.

A needle 8 may be attached to the hub portion 5 of the medical injection device 1. For example, the needle 8 may be glued using a glue 9 to the hub portion 5. The cover of the invention is intended to cover the hub portion 5 of the medical injection device 1, so as to protect the needle 8.

The medical injection device or syringe 1 shown in Figures 1, 2 and 4 may also include a plunger rod (not shown on the figures) having a plunger provided at an end thereof. The plunger is caused to slidably move in the body 2 along an inner surface of the sidewall 3 to cause the medical composition 4 to be expelled from the body 3 through the channel 7 and thus the needle 8. The medical composition 4 comprised in the medical injection device 1 may be for example, a liquid medicament, a drug or a pharmaceutical composition such as a vaccine.

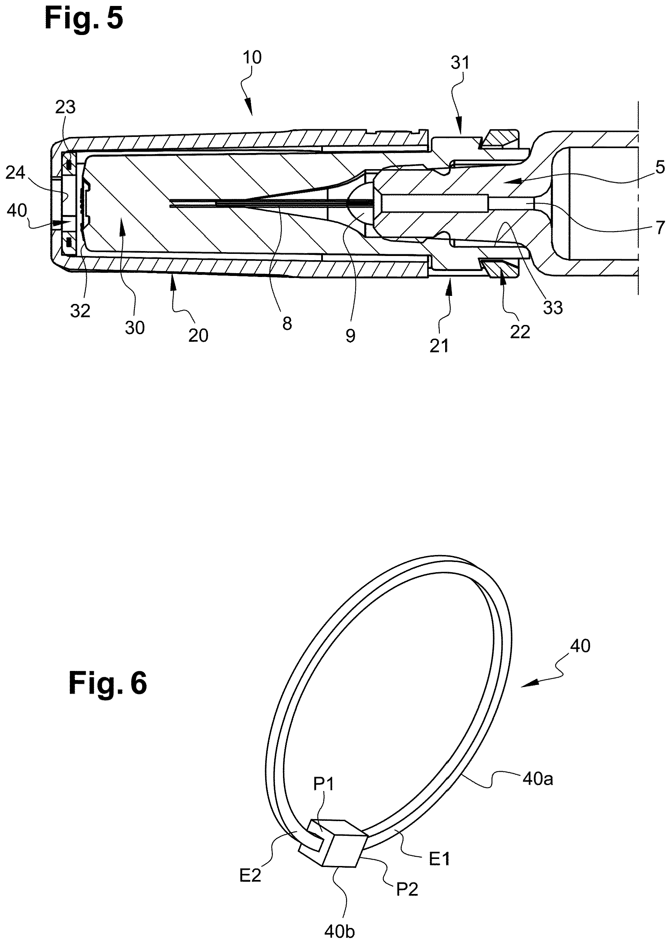

Figures 1 to 5 illustrate a cover 10 according to an emdobiment of the present invention. The cover 10 of the invention is intended to protect the needle 8 mounted on the hub portion 5 of a medical injection device 1. The cover 10 is intended to be mounted on the hub portion 5 of said medical injection device 1. The cover 10 comprises an outer casing 20 and an inner casing 30. The outer casing 20 is typically made of a rigid material while the inner casing 30 wherein the needle 8 is directly engaged when the cover 10 is mounted on the hub portion 6 of the medical injection device 1, is made of a soft material. Preferably, the outer casing 20 is made of polypropylene (PP), polyethylene (PE), polyethylene terephthalate (PET), polystyrene (PS) or polycarbonate (PC), much preferably of polypropylene. Advantageously, the inner casing 30 is made of a deformable material such as TPE (Thermo Plastic Elastomer). Advantageously, the outer casing 20 comprises a partially closed distal end and an opened proximal end. Typically, the distal end of the outer casing comprises a top wall which may comprise at least a through hole 24. Said throug hole 24 may be useful for example for allowing the sterilization gas to enter into said outer casing. Preferably, the inner casing 30 comprises a closed distal end and an opened proximal end.The outer casing 20 can be manufactured so that, at its proximal end, the outer casing 20 comprises at least one locking window 21. Said locking window define a through aperture and is configured to receive at least a radial lug 31 of the inner casing 30. Said radial lug 31 of the inner casing 30 engages with the at least one window 21 of the outer casing 20. Typically the outer casing comprises two locking windows 21 diametricaly opposite. Typically, the inner casing comprises two radial lugs 31 diametrically opposite, or one radial lug extending all aroung the outer surface of the inner casing. The outer casing 20 may comprise abutment portions 22 which are positioned next to locking window 21 allowing to lock the inner casing 20, as the radial lug 31 engages into the locking window 21. The outer casing 20 may be manufactured by over-molding or injection molding.

The outer casing 20 can comprise a top inner wall 23, which may receive a top outer wall 32 of the inner casing 30.

The inner casing 30 may also comprise an inner lateral wall 33 which is intended to at least partially cooperate with the hub portion 5 of the medical injection device 1 when the cover 10 is mounted on said hub portion 5. Said inner lateral wall 33 of the inner casing 30 is intended to sealingly engage an outer surface of the hub portion 5 of the medical injection device 1.

Figures 2 to 6 illustrate a ring-shaped RFID tag 40 that may be embedded between the inner casing 30 and the outer casing 20. For example, the ring-shaped RFID tag 40 may be embedded on a top inner wall 23 of the outer casing 20. Advantageously, the ring-shaped RFID tag 40 at least partially surronds the throug hole 24 comprised in the top wall of the outer casing 20. The ring-shaped RFID tag 40 comprises at least one substantially ring-shaped antenna 40a having two extremities E1, E2. Each extremity E1, E2 can be linked to a portion P1, P2 of the rectangular-shaped RFID chip 40b. During sterilization, the sterilization gas can easily flow through the cover 10 from the opening 24. Indeed, the specific ring-shaped of the RFID tag combined with the radial dimensions of the RFID tag with respect to the cover and the specific position of the RFID tag does not inhibit, during a sterilization step, the sterilization gas going through the outer casing of the cover, in particular the opening. Additionally, the at least one antenna may be as long as desired within a ring conformation. This makes it possible to include a RFID tag in a cover without increasing the overall cover volume. Therefore, this allows increasing data transmission level to the RFID reader.

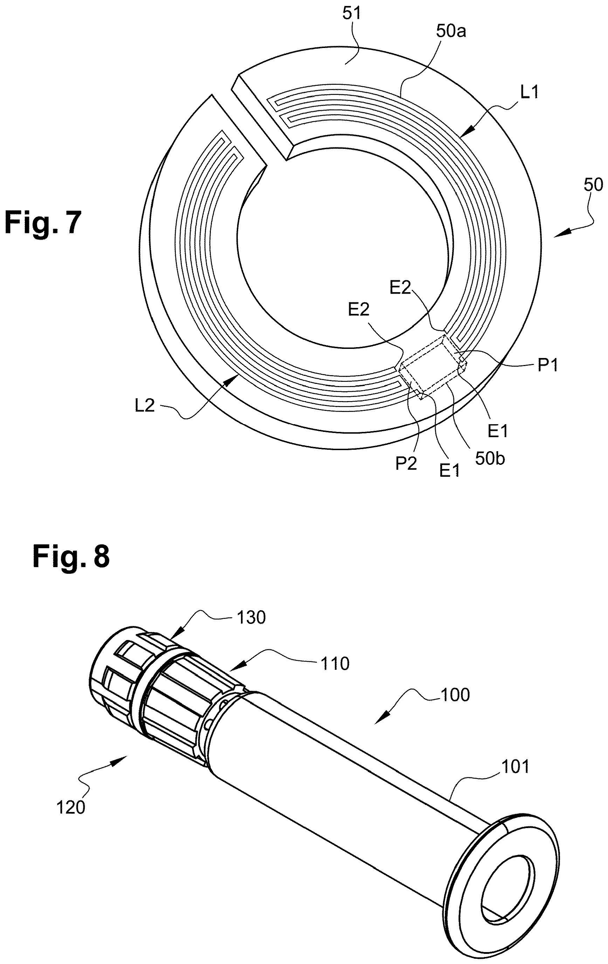

As illustrated in Figure 7, alternatively, the ring-shaped RFID tag 50 can comprise at least one substantially ring-shaped antenna 50a having two legs L1, L2 including respectively a first extremity E1 and a second extremity E2, each first extremity E1 of the legs L1, L2 being linked to a portion P1, P2 of the RFID chip 50b. In this example, the second extremities E2 of the legs L1, L2 of the RFID antenna 50a are not in contact with each other. The ring-shaped RFID tag 50 may comprise one RFID antenna 50a having two legs including a plurality of steady steps. For example, the steady step forms a rectangle shape. For example, the plurality of steady steps have the same amplitude. For example, the RFID chip of the RFID tag 50 is located at equidistance of both RFID antenna extremities. The ring-shaped RFID tag 50 can comprise preferably a substrate 51, for example made of paper or Polyethylene terephthalate (PET).

The ring-shaped tag 40, 50 may be in a form of a dry inlay or wet inlay. When the ring-shaped RFID tags 40, 50 are in a form a wet inlay, the ring-shaped RFID tag are, independently from each other, embedded between a top inner wall 23 of the outer casing 20 and a top outer wall 32 of the inner casing 30.

The ring-shaped tags 40, 50 may be independently from each other LF-RFID tag, HF-RFID tag or HF-RFID tag. Preferably, the ring-shaped tags 40, 50 may be independently from each other a HF-RFID tag.

Nowadays, a RFID tag allows an individual traceability of the medical containers, such as medical injection devices, especially from the manufacturing process until the use in hospital, and preferably until the final user when he is at home. HF-NFC tag are usually used by the final user. In one embodiment, the cover comprises one substantially ring-shaped HF-RFID tag and one HF-NFC tag.

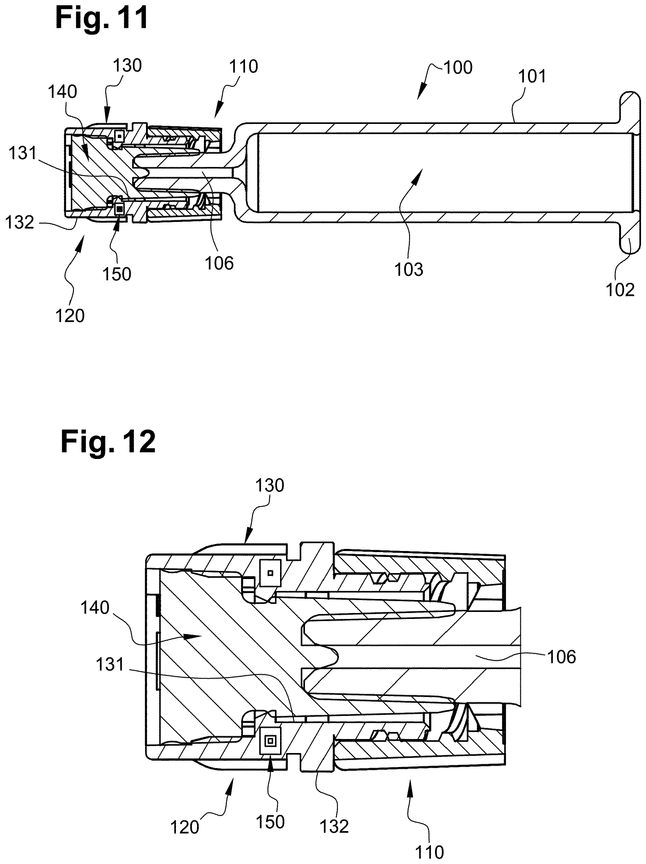

Figures 8, 9, 11 and 13 illustrate a medical injection device 100 comprising a body 101. Said body comprises a sidewall 102 forming a reservoir adapted to contain a medical composition 103 to be injected. The medical injection device 100 further comprises a hub portion 104 provided at its distal end 105. The hub portion 104 is partially hollow so as to form a channel 106 in fluidic communication with the body 101.

An adaptor 110 is intended to be mounted on the distal end 105 of the medical injection device 100, more precisely onto an outer surface of the hub portion 104 of the medical injection device 100.

The adaptor 110 is configured to be secured to the medical container 100 at its proximal end, and may be in the form of a connecting ring at its distal end, configured to receive a cover 120 according to the present invention. In practice, to use the medical injection device 100, the cover 120 is removed from the adaptor 110 and a connector (not shown), such as a needle hub or an intravenous line, is mounted on the adaptor 110 in order to establish a fluidic communication between a passageway 106 of the medical container 100 and said connector.

Figures 8 to 13 illustrate a cover 120 according to an emdobiment of the present invention. The cover 120 of the invention is intended to ensure the sterility and the integrity of a medical injection device 100. The cover 120 is intended to be mounted on the adaptator 110. The cover 120 comprises an outer casing 130 and an inner casing 140. The outer casing 130 is typically made of a rigid material while the inner casing 140 is made of a soft material. Preferably, the outer casing 130 is made of polypropylene (PP), polyethylene (PE), polyethylene terephthalate (PET), polystyrene (PS) or polycarbonate (PC), much preferably of polypropylene. Advantageously, the inner casing 140 is made of a deformable material such as TPE (Thermo Plastic Elastomer). Preferably, the outer casing 130 comprises a closed distal end and an opened proximal end. Typically, the inner casing 140 comprises a closed distal end and an opened proximal end. The outer casing 130 is configured to receive the inner casing 140.

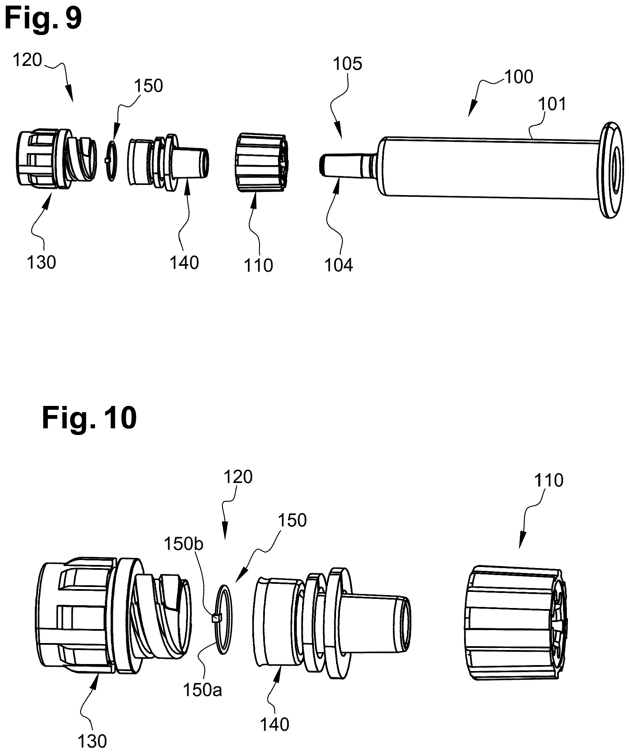

Figures 9 to 12 illustrate one embodiment wherein the ring-shaped RFID tag 150 is embedded within the cover 120. The ring-shaped RFID tag 150 is embedded between a lateral inner wall 131 of the outer casing 130 and a lateral outer wall 132 of the outer casing 130.

As illustrated in Figures 9 to 12, the ring-shaped RFID tag 150 may be embedded at the distal ends of the outer 130 and inner casings 140. The ring-shaped RFID tag 150 may comprise at least one antenna 150a having two extremities E1, E2. Each extremity E1, E2 can be linked to a portion P1, P2 of the RFID chip 150b.

Alternatively, the ring-shaped RFID tag 150 may be embedded at the proximal ends of the outer 130 and inner casings 140.

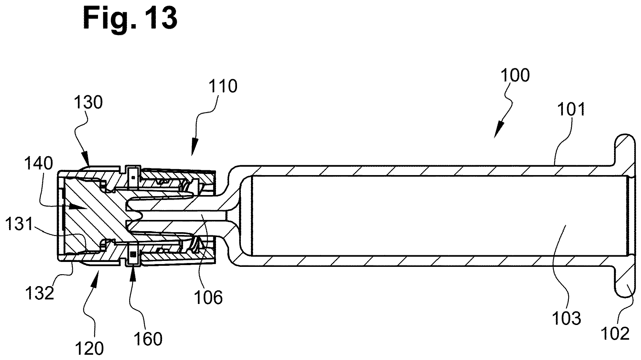

Figure 13 illustrates an embodiment wherein the ring-shaped RFID tag 160 may be embedded at a middle portion of the outer casing 130 and in particular, within a middle portion of the outer casing 130. In this example, the ring-shaped RFID tag 160 is located within the outer casing 130 and is flush with a lateral inner wall 131 of the outer casing 130.

For example, the ring-shaped RFID tag 160 may comprise at least one antenna having two legs including respectively a first extremity and a second extremity, each first extremity of the legs being linked to a portion of the RFID chip and each second extremity including a plurality of sub-legs (not shown). In this Example, the two legs are respectively in a form of a rake (not shown). For example, the RFID chip is located at equidistance of both RFID antenna extremities.

The ring-shaped tags 150, 160 may be in a form of a dry inlay or wet inlay. When the ring-shaped RFID tags 150, 160 are in a form a wet or dry inlay, the ring-shaped RFID tags 150, 160 are respectively embedded between a lateral inner wall 131 of the outer casing 130 and a lateral outer wall 132 of the outer casing 130 by overmolding.

The ring-shaped tags 150, 160 may be independently from each other LF-RFID tag, HF-RFID tag or HF-RFID tag. Preferably, the ring-shaped tags 150, 160 may be independently from each other a HF-RFID tag.

The cover according to the present invention allows a long distance highly effective individual identification of a medical injection device with no impact on visual inspection, with few or no risks of being removed or damaged, and with a limited impact on the manufacturing process.

## Claims

1. Cover (10, 120) for a medical injection device (1, 100), said medical injection device (1, 100) having a hub portion (5, 104) defined at its distal end, the said cover (10, 120) comprising:
- an outer casing (20, 130) formed of a first material, said outer casing (20, 130) having a distal end and a proximal end,
- an inner casing (30, 140) formed of a second material different from the said first material and defining a cavity capable of receiving in a sealing way, at least part of the hub portion (5, 104), said inner casing (30, 140) having a distal end and a proximal end, and being at least partly inserted within the outer casing (20,130) and at least partially in contact with the outer casing (20, 130) and wherein the said cover is **characterised by** comprising at least one substantially ring-shaped Radio Frequency Identification RFID tag (40, 50, 150, 160) embedded within the cover (10, 120), said ring-shaped RFID tag (40, 50, 150, 160) comprising a RFID chip (40b, 50b, 150b) connected to at least one RFID ring-shaped antenna (40a, 50a, 150a).

2. Cover (10, 120) for a medical injection device according to claim 1, wherein the ring-shaped RFID tag is located between the outer casing and the inner casing.

3. Cover (10, 120) for a medical injection device according to claim 1 or 2, wherein the ring-shaped RFID tag (40, 50, 150, 160) is located at the distal end of the outer (20, 130) and inner casings (30, 140), at a middle portion of the outer casing (20, 130) or at the proximal end of the outer casing (20, 130) and inner casings (30, 140).

4. Cover (10, 120) for a medical injection device according to any of claims 1 to 3, wherein the ring-shaped RFID tag (40, 50, 150, 160) is located on a top inner wall (23) of the outer casing (20, 130) or on a top outer wall (32) of the inner casing (30, 140).

5. Cover (10, 120) for a medical injection device according to any of claims 1 to 3, wherein the ring-shaped RFID tag (40, 50, 150, 160) is located between a top inner wall (23) of the outer casing (20, 130) and a top outer wall (32) of the inner casing (30, 140).

6. Cover (10, 120) for a medical injection device according to any of claims 1 to 3, wherein the ring-shaped RFID tag (40, 50, 150, 160) is embedded within the outer casing, especially between a lateral inner wall (131) of the outer casing (20, 130) and a lateral outer wall (132) of the outer casing (20, 130).

7. Cover (10, 120) for a medical injection device according to claim 6, wherein the ring-shaped RFID tag (40, 50, 150, 160) is located within the outer casing (20, 130) and is flush with a lateral inner wall (131) of the outer casing (20, 130).

8. Cover (10, 120) for a medical injection device according to any of claims 1 to 7, wherein the ring-shaped RFID tag (40, 50, 150, 160) is an Ultra High Frequency Radio Frequency Identification UHF-RFID tag, a High Frequency Radio Frequency Identification HF-RFID tag, a Low Frenquency Radio Indentification LF-RFID tag, a High-Frequency Near Field Communication HF-NFC tag or a double frequency tag including simultaneously a HF-NFC and an UHF RFID.

9. Cover (10, 120) for a medical injection device according to any of claims 1 to 8, wherein the ring-shaped RFID tag (40, 50, 150, 160) comprises at least one antenna (40a, 50a, 150a) having two extremities (E1, E2), each extremity (E1, E2) being linked to a portion (P1, P2) of the RFID chip (40b, 50b, 150b).

10. Cover (10, 120) for a medical injection device according to any of claims 1 to 8, wherein the ring-shaped RFID tag (40, 50, 150, 160) comprises at least one antenna (40a, 50a, 150a) having two legs (L1, L2) including respectively a first extremity (E1) and a second extremity (E2), each first extremity (E1) of the legs (L1, L2) being linked to a portion (P1, P2) of the RFID chip (40b, 50b, 150b).

11. Cover (10, 120) for a medical injection device according to any of claims 1 to 8, wherein the ring-shaped RFID tag (40, 50, 150, 160) comprises at least one antenna (40a, 50a, 150a) having two legs (L1, L2) including respectively a first extremity (E1) and a second extremity (E2), each first extremity (E1) of the legs (L1, L2) being linked to a portion (P1, P2) of the RFID chip (40b, 50b, 150b) and each second extremity (E2) including a plurality of sub-legs.

12. Medical injection device (1, 100) comprising a hub portion (5, 104), and a cover (10, 120) according to any of claims 1 to 11.

13. Method for manufacturing a cover (10, 120) comprising an inner casing (30, 140) and an outer casing (20, 130) according to any of claims 1 to 11, comprising:
A. Providing the inner casing (30, 140) and the outer casing (20, 130), said inner casing (30, 140) defining a cavity capable of receiving in a sealing way at least part of a hub portion (5, 104) defined at the distal end of a medical injection device (1, 100),
B. Providing the inner casing (30, 140) and/or the outer casing (20, 130) with at least one a ring-shaped RFID tag(40, 50, 150, 160), said ring-shaped RFID tag (40, 50, 150, 160) comprising a RFID chip (40b, 50b, 150b) connected to at least one ring-shaped RFID antenna (40a, 50a, 150a) and
C. Assembling the cover (10, 120) by inserting the inner casing (30, 140) within the outer casing (20, 130) so that the RFID tag (40, 50, 150, 160) is embedded within the cover (10, 120).

14. Method according to claim 13, wherein the ring-shaped RFID tag (40, 50, 150, 160) is implemented within the cover (10, 120) by overmolding, by adhesive bonding or by assembly.

## Patentansprüche

1. Abdeckung (10, 120) für eine medizinische Injektionsvorrichtung (1, 100), wobei die medizinische Injektionsvorrichtung (1, 100) einen an ihrem distalen Ende definierten Ansatzabschnitt (5, 104) aufweist, die Abdeckung (10, 120) umfassend:
- ein äußeres Gehäuse (20, 130), das aus einem ersten Material gebildet ist, wobei das äußere Gehäuse (20, 130) ein distales Ende und ein proximales Ende aufweist,
- ein inneres Gehäuse (30, 140), das aus einem zweiten Material gebildet ist, das sich von dem ersten Material unterscheidet, und das einen Hohlraum definiert, der in der Lage ist, mindestens einen Teil des Ansatzabschnitts (5, 104) auf dichtende Art und Weise zu empfangen, wobei das innere Gehäuse (30, 140) ein distales Ende und ein proximales Ende aufweist und mindestens teilweise in das äußere Gehäuse (20, 130) eingesetzt ist und mindestens teilweise in Kontakt mit dem äußeren Gehäuse (20, 130) eingefügt ist und wobei die Abdeckung **dadurch gekennzeichnet ist, dass** sie mindestens einen im Wesentlichen ringförmigen Radiofrequenzidentifikations-RFID-Tag (40, 50, 150, 160) umfasst, der in die Abdeckung (10, 120) eingebettet ist, wobei der ringförmige RFID-Tag (40, 50, 150, 160) einen RFID-Chip (40b, 50b, 150b) umfasst, der mit mindestens einer ringförmigen RFID-Antenne (40a, 50a, 150a) verbunden ist.

2. Abdeckung (10, 120) für eine medizinische Injektionsvorrichtung nach Anspruch 1, wobei der ringförmige RFID-Tag zwischen dem äußeren Gehäuse und dem inneren Gehäuse angeordnet ist.

3. Abdeckung (10, 120) für eine medizinische Injektionsvorrichtung nach Anspruch 1 oder 2, wobei der ringförmige RFID-Tag (40, 50, 150, 160) am distalen Ende des äußeren (20, 130) und des inneren Gehäuses (30, 140), an einem mittleren Abschnitt des äußeren Gehäuses (20, 130) oder am proximalen Ende des äußeren Gehäuses (20, 130) und des inneren Gehäuses (30, 140) angeordnet ist.

4. Abdeckung (10, 120) für eine medizinische Injektionsvorrichtung nach einem der Ansprüche 1 bis 3, wobei der ringförmige RFID-Tag (40, 50, 150, 160) an einer oberen Innenwand (23) des äußeren Gehäuses (20, 130) oder an einer oberen Außenwand (32) des inneren Gehäuses (30, 140) angeordnet ist.

5. Abdeckung (10, 120) für eine medizinische Injektionsvorrichtung nach einem der Ansprüche 1 bis 3, wobei der ringförmige RFID-Tag (40, 50, 150, 160) zwischen einer oberen Innenwand (23) des äußeren Gehäuses (20, 130) und einer oberen Außenwand (32) des inneren Gehäuses (30, 140) angeordnet ist.

6. Abdeckung (10, 120) für eine medizinische Injektionsvorrichtung nach einem der Ansprüche 1 bis 3, wobei der ringförmige RFID-Tag (40, 50, 150, 160) in das äußere Gehäuse eingebettet ist, insbesondere zwischen einer seitlichen Innenwand (131) des äußeren Gehäuses (20, 130) und einer seitlichen Außenwand (132) des äußeren Gehäuses (20, 130).

7. Abdeckung (10, 120) für eine medizinische Injektionsvorrichtung nach Anspruch 6, wobei der ringförmige RFID-Tag (40, 50, 150, 160) innerhalb des äußeren Gehäuses (20, 130) angeordnet ist und mit einer seitlichen Innenwand (131) des äußeren Gehäuses (20, 130) bündig ist.

8. Abdeckung (10, 120) für eine medizinische Injektionsvorrichtung nach einem der Ansprüche 1 bis 7, wobei der ringförmige RFID-Tag (40, 50, 150, 160) ein Ultrahochfrequenz-Radiofrequenzidentifikations-Tag (UHF-RFID), ein Hochfrequenz-Radiofrequenzidentifikations-Tag (HF-RFID), ein Niederfrequenz-Radioidentifikations-Tag (LF-RFID), ein Hochfrequenz-Nahfeldkommunikations-Tag (HF-NFC) oder ein Doppelfrequenz-Tag ist, der gleichzeitig ein HF-NFC und ein UHF-RFID enthält.

9. Abdeckung (10, 120) für eine medizinische Injektionsvorrichtung nach einem der Ansprüche 1 bis 8, wobei der ringförmige RFID-Tag (40, 50, 150, 160) mindestens eine Antenne (40a, 50a, 150a) mit zwei Extremitäten (E1, E2) aufweist, wobei jede Extremität (E1, E2) mit einem Abschnitt (P1, P2) des RFID-Chips (40b, 50b, 150b) verbunden ist.

10. Abdeckung (10, 120) für eine medizinische Injektionsvorrichtung nach einem der Ansprüche 1 bis 8, wobei der ringförmige RFID-Tag (40, 50, 150, 160) mindestens eine Antenne (40a, 50a, 150a) mit zwei Schenkeln (L1, L2) einschließlich eines ersten Endes (E1) bzw. eines zweiten Endes (E2) umfasst, wobei jedes erste Ende (E1) der Schenkel (L1, L2) mit einem Abschnitt (P1, P2) des RFID-Chips (40b, 50b, 150b) verbunden ist.

11. Abdeckung (10, 120) für eine medizinische Injektionsvorrichtung nach einem der Ansprüche 1 bis 8, wobei der ringförmige RFID-Tag (40, 50, 150, 160) mindestens eine Antenne (40a, 50a, 150a) umfasst, die zwei Schenkel (L1, L2) aufweist, die jeweils ein erstes Ende (E1) und ein zweites Ende (E2) einschließen, wobei jedes erste Ende (E1) der Schenkel (L1, L2) mit einem Abschnitt (P1, P2) des RFID-Chips (40b, 50b, 150b) verbunden ist und jedes zweite Ende (E2) eine Vielzahl von Unterschenkeln einschließt.

12. Medizinische Injektionsvorrichtung (1, 100), umfassend einen Ansatzabschnitt (5, 104) und eine Abdeckung (10, 120) nach einem der Ansprüche 1 bis 11.

13. Verfahren zur Herstellung einer Abdeckung (10, 120), umfassend ein inneres Gehäuse (30, 140) und ein äußeres Gehäuse (20, 130) nach einem der Ansprüche 1 bis 11, umfassend:
A. Bereitstellen des inneren Gehäuses (30, 140) und des äußeren Gehäuses (20, 130), wobei das innere Gehäuse (30, 140) einen Hohlraum definiert, der in der Lage ist, mindestens einen Teil eines am distalen Ende einer medizinischen Injektionsvorrichtung (1, 100) definierten Ansatzabschnittes (5, 104) auf dichte Art zu empfangen,
B. Bereitstellen des inneren Gehäuses (30, 140) und/oder des äußeren Gehäuses (20, 130) mit mindestens einem ringförmigen RFID-Tag (40, 50, 150, 160), wobei der ringförmige RFID-Tag (40, 50, 150, 160) einen RFID-Chip (40b, 50b, 150b) umfasst, der mit mindestens einer ringförmigen RFID-Antenne (40a, 50a, 150a) verbunden ist und
C. Zusammensetzen der Abdeckung (10, 120) durch Einsetzen des inneren Gehäuses (30, 140) in das äußere Gehäuse (20, 130), sodass der RFID-Tag (40, 50, 150, 160) in die Abdeckung (10, 120) eingebettet wird.

14. Verfahren nach Anspruch 13, wobei der ringförmige RFID-Tag (40, 50, 150, 160) innerhalb der Abdeckung (10, 120) durch Umformen, durch Kleben oder durch Zusammensetzen implementiert wird.

## Revendications

1. Couvercle (10, 120) pour un dispositif d'injection médical (1, 100), ledit dispositif d'injection médical (1, 100) ayant une partie de raccord (5, 104) définie au niveau de son extrémité distale, ledit couvercle (10, 120) comprenant :
- un boîtier externe (20, 130) formé d'un premier matériau, ledit boîtier externe (20, 130) ayant une extrémité distale et une extrémité proximale,
- un boîtier interne (30, 140) formé d'un second matériau différent dudit premier matériau et définissant une cavité capable de recevoir de manière étanche, au moins une portion de la partie de raccord (5, 104), ledit boîtier interne (30, 140) ayant une extrémité distale et une extrémité proximale, et étant au moins partiellement inséré à l'intérieur du boîtier externe (20, 130) et au moins partiellement en contact avec le boîtier externe (20, 130) et
dans lequel ledit couvercle est **caractérisé en ce qu'**il comprend au moins une étiquette d'identification par Radiofréquence (RFID) sensiblement en forme d'anneau (40, 50, 150, 160) intégrée dans le couvercle (10, 120), ladite étiquette RFID en forme d'anneau (40, 50, 150, 160) comprenant une puce RFID (40b, 50b, 150b) reliée à au moins une antenne RFID en forme d'anneau (40a, 50a, 150a).

2. Couvercle (10, 120) pour un dispositif d'injection médical selon la revendication 1, dans lequel l'étiquette RFID en forme d'anneau est située entre le boîtier externe et le boîtier interne.

3. Couvercle (10, 120) pour un dispositif d'injection médical selon la revendication 1 ou 2, dans lequel l'étiquette RFID en forme d'anneau (40, 50, 150, 160) est située au niveau de l'extrémité distale des boîtiers externe (20, 130) et interne (30, 140), au niveau d'une partie intermédiaire du boîtier externe (20, 130) ou au niveau de l'extrémité proximale du boîtier externe (20, 130) et des boîtiers internes (30, 140).

4. Couvercle (10, 120) pour un dispositif d'injection médical selon l'une des revendications 1 à 3, dans lequel l'étiquette RFID en forme d'anneau (40, 50, 150, 160) est située sur une paroi interne supérieure (23) du boîtier externe (20, 130) ou sur une paroi externe supérieure (32) du boîtier interne (30, 140).

5. Couvercle (10, 120) pour un dispositif d'injection médical selon l'une des revendications 1 à 3, dans lequel l'étiquette RFID en forme d'anneau (40, 50, 150, 160) est située entre une paroi interne supérieure (23) du boîtier externe (20, 130) et une paroi externe supérieure (32) du boîtier interne (30, 140).

6. Couvercle (10, 120) pour un dispositif d'injection médical selon l'une des revendications 1 à 3, dans lequel l'étiquette RFID en forme d'anneau (40, 50, 150, 160) est intégrée dans le boîtier externe, en particulier entre une paroi interne latérale (131) du boîtier externe (20, 130) et une paroi externe latérale (132) du boîtier externe (20, 130).

7. Couvercle (10, 120) pour un dispositif d'injection médical selon la revendication 6, dans lequel l'étiquette RFID en forme d'anneau (40, 50, 150, 160) est située à l'intérieur du boîtier externe (20, 130) et affleure une paroi interne latérale (131) du boîtier externe (20, 130).

8. Couvercle (10, 120) pour un dispositif d'injection médical selon l'une des revendications 1 à 7, dans lequel l'étiquette RFID en forme d'anneau (40, 50, 150, 160) est une étiquette d'identification par Radiofréquence à Ultra Haute Fréquence (UHF-RFID), une étiquette d'identification par Radiofréquence à Haute Fréquence (HF-RFID), une étiquette d'identification par Radiofréquence à Basse Fréquence (LF-RFID), une étiquette de Communication en Champ Proche à Haute Fréquence (HF-NFC) ou une étiquette à double fréquence comprenant simultanément une HF-NFC et une UHF-RFID.

9. Couvercle (10, 120) pour un dispositif d'injection médical selon l'une des revendications 1 à 8, dans lequel l'étiquette RFID en forme d'anneau (40, 50, 150, 160) comprend au moins une antenne (40a, 50a, 150a) ayant deux extrémités (E1, E2), chaque extrémité (E1, E2) étant liée à une partie (P1, P2) de la puce RFID (40b, 50b, 150b).

10. Couvercle (10, 120) pour un dispositif d'injection médical selon l'une des revendications 1 à 8, dans lequel l'étiquette RFID en forme d'anneau (40, 50, 150, 160) comprend au moins une antenne (40a, 50a, 150a) ayant deux branches (L1, L2) comprenant respectivement une première extrémité (E1) et une seconde extrémité (E2), chaque première extrémité (E1) des branches (L1, L2) étant liée à une partie (P1, P2) de la puce RFID (40b, 50b, 150b).

11. Couvercle (10, 120) pour un dispositif d'injection médical selon l'une des revendications 1 à 8, dans lequel l'étiquette RFID en forme d'anneau (40, 50, 150, 160) comprend au moins une antenne (40a, 50a, 150a) ayant deux branches (L1, L2) comprenant respectivement une première extrémité (E1) et une seconde extrémité (E2), chaque première extrémité (E1) des branches (L1, L2) étant liée à une partie (P1, P2) de la puce RFID (40b, 50b, 150b) et chaque seconde extrémité (E2) comprenant une pluralité de sous-branches.

12. Dispositif d'injection médical (1, 100) comprenant une partie de raccord (5, 104) et un couvercle (10, 120) selon l'une des revendications 1 à 11.

13. Procédé de fabrication d'un couvercle (10, 120) comprenant un boîtier interne (30, 140) et un boîtier externe (20, 130) selon l'une des revendications 1 à 11, comprenant :
A. la fourniture du boîtier interne (30, 140) et du boîtier externe (20, 130), ledit boîtier interne (30, 140) définissant une cavité capable de recevoir de manière étanche, au moins une portion d'une partie de raccord (5, 104) définie au niveau de l'extrémité distale d'un dispositif d'injection médical (1, 100),
B. la fourniture au boîtier interne (30, 140) et/ou au boîtier externe (20, 130) d'au moins une étiquette RFID en forme d'anneau (40, 50, 150, 160), ladite étiquette RFID en forme d'anneau (40, 50, 150, 160) comprenant une puce RFID (40b, 50b, 150b) reliée à au moins une antenne RFID en forme d'anneau (40a, 50a, 150a) et
C. l'assemblage du couvercle (10, 120) en insérant le boîtier interne (30, 140) dans le boîtier externe (20, 130) de sorte que l'étiquette RFID (40, 50, 150, 160) soit intégrée dans le couvercle (10, 120).

14. Procédé selon la revendication 13, dans lequel l'étiquette RFID en forme d'anneau (40, 50, 150, 160) est mise en oeuvre dans le couvercle (10, 120) par surmoulage, par collage ou par assemblage.
